# EUROPÄISCHE PATENTANMELDUNG

(11) **EP 0 621 240 A1**
(43) Veröffentlichungstag der Anmeldung: **26.10.1994**
(21) Anmeldenummer: 94104857.1
(22) Anmeldetag: 28.03.1994
(51) Int. Cl.: C02F 1/72, C07D 209/48

(54) **Biozidfreie Geruchsbekämpfung in Abwässern**

(30) Priorität: 20.04.1993 DE 4312734
(71) Anmelder: BK LADENBURG GmbH, Gesellschaft für chemische Erzeugnisse, D-68526 Ladenburg (DE)
(72) Erfinder: Krämer, Albrecht, Dr., D-69120 Heidelberg (DE); Salzburger, Wolfram, Dr., D-64625 Bensheim (DE); Stoffel, Erwin, D-69514 Laudenbach (DE); Steudel, Elke, D-67269 Grünstadt (DE)
(74) Vertreter: Grussdorf, Jürgen, Dr.

(57) **Zusammenfassung**

Die vorliegende Erfindung betrifft ein Verfahren zur biozitfreien Geruchsbekämpfung in Abwässern, wobei man dem Abwasser eine Phthalimidoalkylperoxicarbonsäure der Formel I, 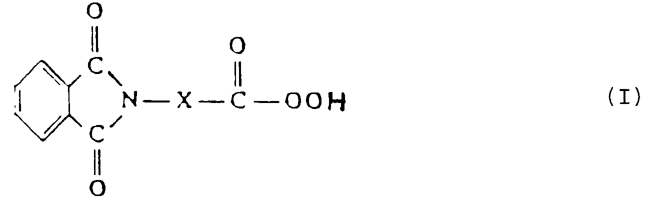
worin x eine Alkylengruppe mit 4 bis 10 Kohlenstoffen bedeutet,
in einer Konzentration von 0,1 bis 10 g/kg Abwasser zufügt.

## Beschreibung

Die vorliegende Erfindung betrifft ein neues Verfahren zur biozitfreien Geruchsbekämpfung in Abwässern, insbesondere Prozeßabwässern der Papierindustrie und verwandter Bereiche, durch Zusatz von Imidoperoxicarbonsäuren.

Bei der Abwasseraufbereitung, Lagerung, Beseitigung oder Bearbeitung zur Wiederverwendung von Abfallstoffen von verschiedenen technischen Prozessen, treten geruchsbelästigende Stoffe auf. Außer den aus dem technischen Prozeß selbst stammenden Produkten entstehen die Geruchsimmissionen aus Kläranlagen, durch den bakteriellen Abbau organischer Verunreinigungen des Abwassers, hauptsächlich von Eiweißkörpern und Kohlehydraten. Den wesentlichen Anteil der Immissionen bilden Schwefelverbindungen, daneben Stickstoffverbindungen, während Sauerstoffverbindungen, aufgrund der erheblich höheren Geruchsschwellenwerte, weniger störend sind. Die schwefel- und stickstoffhaltigen Verbindungen stammen vornehmlich aus den zum Teil schwefelhaltigen Aminosäuren der Eiweißkörper. Bakterien bewirken, sowohl bei aeroben, wie auch im besonderen bei anaeroben Fäulnisprozessen, die Bildung von Schwefelwasserstoff und seinen organischen Derivaten, sowie niederen aliphatischen und aromatischen Aminen wie auch von zahlreichen anderen Derivaten mit Schwefel- und Stickstoffhaltigen funktionellen Gruppen. Besonders hohe Abwasserbelastungen dieser Art verursachen Anlagen der Papierindustrie, Massentierhaltungen und Futtermittelfabriken, welche deshalb häufig über eigene Kläranlagen verfügen.

Die der Kläranlage zugeführten Abwässer müssen von dieser so aufbereitet werden, daß das gereinigte Wasser die behördlichen Auflagen, Anforderungen bezüglich der Bestimmungen über Schadstoffgrenzwerte, Salzfracht, Sauerstoffgehalt, pH-Wert usw. genügen. Für diese Reinigung genügt im allgemeinen eine mechanische und zweistufige biologische und bakterielle Reinigung, welche im allgemeinen in Form einer anaeroben und einer aeroben Stufe durchgeführt wird. Problematisch bei diesen Prozessen ist aber, wie gesagt, die gleichzeitige Bildung entsprechender Faulgase und die damit verbundenen Geruchsimmissionen.

Zur Bekämpfung der Geruchsimmissionen aus Abwässern sind bereits verschiedene Verfahren bekannt. Beispielhaft seien folgende genannt:
1. Zusatz von Zinksalzen organischer Säuren. Durch die Zinkionen werden die sulfidischen Verbindungen als Zinkkomplexe gebunden und durch die Carbonsäuren gleichzeitig die Amine als Salze gebunden, so daß beide Komponenten weniger stark riechend werden. Nachteilig an diesem Verfahren ist einerseits, daß die zugesetzten Zinkverbindungen eine zusätzliche Abwasserbelastung darstellen und andrerseits, insbesondere die geruchsbildenden Amine nicht ausreichend fixiert werden (DD-PS 37 26 636)
2. Zusatz von Wasserstoffperoxid, Natriumperoxid oder Natriumpercarbonat. Durch Spontanzerfall gebildete Hydroxylradikale sind wirkungsvolle Oxidationsmittel für die sulfidischen und teilweise auch die stickstoffhaltigen Verbindungen. Als nachteilig erweist sich, daß diese Verbindungen als am Eintrittsort bereits weitgehend zerfallen und relativ starke Oxidationsmittel auch andere organische Verbindungen oxidieren, so daß ein relativ großer Überschuß eingesetzt werden muß und andererseits die Verbindungen biotoxisch sind und damit erst nach den biologischen Klärverfahren angewendet werden können (US-PS 4,263,136).
3. Zusatz niederer aliphatischer Persäuren. Diese Verbindungen sind zwar relativ preiswert zu erhalten und auch wirksame Oxidationsmittel, jedoch weisen die durch Reduktion entstehenden aliphatischen Säuren selbst einen störenden Eigengeruch auf. Darüberhinaus sind diese Persäuren nicht beständig und besonders in konzentrierten wässrigen Lösungen explosiv, so daß sie nur mit erheblichen Vorsichtsmaßnahmen gehandhabt werden können.
4. Dibenzoylperoxid bzw. Perbenzoesäure. Dibenzoylperoxid erfordert die Verwendung von entsprechenden Radikalstartern. Beide Verbindungen besitzen den Nachteil, daß mit dem Einbringen der aromatischen Grundkörper wiederum schwer abbaubare Produkte dem Abwasser zugeführt werden.
5. Aktivkohle. Aktivkohlefilter haben zwar den Vorteil relativ breit ein großes Spektrum an Verbindungen zu absorbieren, jedoch ist insbesondere gegen die geruchswirksamen niedermolekularen Verbindungen die Absorptionskapazität relativ gering. Darüberhinaus ist die Wiederaufarbeitung von Aktivkohlefiltern schwierig und eine Entsorgung der Kohlefilter aus wirtschaftlichen Gründen nicht tragbar (DD-PS 22 05 978)
6. Zusatz von Persulfaten. Persulfate sind ebenfalls als geruchsbeseitigende Mittel eingesetzt worden, haben jedoch den Nachteil, daß sie als sehr starke Oxidationsmittel wiederum biozit sind und außer den geruchsbildenden Stoffen auch andere organische Substanzen oxidieren, so daß sie in größeren Mengen eingesetzt werden müssen. Darüberhinaus belasten die entstehenden Sulfate das Abwasser (DD-PS 41 32 867)
Es bestand daher die Aufgabe, neue Mittel zu finden, mit denen sich Geruchsimmissionen in Abwässern wirksam bekämpfen lassen, ohne daß diese biozit wirken oder daraus selbst geruchsbildende oder abwasserbelastende Stoffe entstehen.

Diese Aufgabe wird durch die Merkmale des Hauptanspruchs gelöst und durch die Merkmale der Unteransprüche gefördert.

Imidoperoxicarbonsäuren, insbesondere die erfindungsgemäßen Phthalimidoalkylperoxicarbonsäuren der Formel I 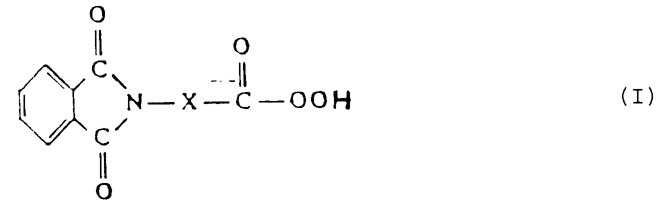
worin x eine Alkylengruppe mit 4 bis 10 Kohlenstoffen bedeutet
sind beispielsweise aus der DE-OS 38 23 172 bekannt. Lagerstabile Granulate und Formulierungen solcher Verbindungen sind aus der DE-OS 40 12 769A1 und EP 0 435 397A2 und WO 90 07 501 bekannt. Gemäß diesen Patentschriften sollen diese Verbindungen als Bleich-, Oxidations- und Desinfektionsmittel in Waschmitteln verwendet werden (vgl. auch JP 04-103 698, JP 04-103 697, EP 442 549A2).

Aus der US 51 32 431 A ist ferner bekannt, diese Verbindungen durch Reaktion von Phthalimidocarbonsäuren mit Wasserstoffperoxid in einem statischen Mischer herzustellen und als Oxidationsmittel in der organischen Synthese oder als Bleichmittel in Waschmitteln zu verwenden.

Überraschenderweise hat sich nunmehr herausgestellt, daß diese Verbindungen in geringen Dosierungen im Stande sind, im Abwasser relativ selektiv die enthaltenen sulfidischen Verbindungen, welche die Hauptmenge der Geruchsbelastung darstellen, zu oxidieren, wobei nicht mehr flüchtige Sulphonsäuren und Sulfate entstehen, ohne die in erheblichem Überschuß in den Abwässern enthaltenen sonstigen organischen Produkte in größerem Ausmaß zu oxidieren. Aus diesem Grund können relativ geringe Mengen wirksam eingesetzt werden. Es ist weiterhin überraschend, daß in diesen Konzentrationen die Persäuren nicht oder nur wenig biozit wirken, so daß der Wirkstoff beispielsweise der Vorklärstufe oder dem Abwasser der anaeroben Reinigungsstufe zugefügt werden kann.

Wie die beigefügte Tabelle 1 zeigt, bei der einem stark faulig riechenden Abwasser aus einer Pappefabrikation verschiedene Mengen von Phthalimidohexanperoxicarbonsäure zugesetzt wurden, wird bereits bei einer Wirkstoffkonzentration von nur 0,2 g/kg Abwasser im Verlauf von 2 Stunden die überwiegende Menge der den Geruch bildenden Sulfide beseitigt, während bei kürzeren Einwirkungszeiten (15 Minuten) bzw. noch geringeren Konzentrationen ein starker Faulgeruch beobachtet wird. Eine noch höhere Konzentration von 1 g/kg weist nach nur 15minütiger Einwirkungszeit bereits nur noch einen schwachen Geruch auf und nach Verlauf von 2 Stunden ist der Geruch praktisch verschwunden. Das Gleiche wird bei Konzentrationen von 10 g/kg beobachtet, wo der Geruch bereits nach 15 Minuten nicht mehr wahrnehmbar ist.

Die wirksame Konzentration der erfindungsgemäßen Phthalimidoperoxicarbonsäuren liegt daher bei 0,1 bis 10 g/kg, vorzugsweise bei etwa 0,5 bis 5 g/kg, insbesondere aber bei etwa 1 bis 2 g/kg Abwasser. Bei besonders stark belasteten Abwässern sind selbstverständlich höhere Konzentrationen anzuwenden, bei wenig verschmutzten Abwässern ist ggf. mit einer geringeren Konzentration auszukommen. Aus wirtschaftlichen Gründen und wegen des vergleichsweise langsamen Zerfalls der Peroxisäuren in Abwesenheit entsprechender Reduktionsmittel, sind größere Überschüsse des Wirkstoffs zu vermeiden.

In der beigefügten Figur 1 ist die Aktivsauerstoffkonzentration in einem Prozeßabwasser, wie es auch dem Versuch in der Tabelle 1 zugrunde liegt, untersucht worden. Es wurde dabei eine Konzentration von 1 g/kg ursprünglich vorgelegt. Wie die Verlaufskurve zeigt, wird zunächst in einer sehr raschen Reaktion etwa 30 % des Wirkstoffes abgebaut, wodurch offensichtlich die freien SH-Gruppen oxidiert werden. Anschließend wird in einer weiteren, wesentlich langsameren Reaktion, die über viele Stunden verläuft, der Restperoxidgehalt langsam abgebaut. Es wird angenommen, daß diese langsame Nachreaktion auf der Oxidation der durch mikrobielle Gärung nachgebildeten Sulfide, einem langsamen spontanen Zerfall der Peroxicarbonsäure durch katalytisch wirkende Verunreinigungen und letztlich auf einer langsamen Oxidation anderer Bestandteile der Lösung beruht. In welchem Ausmaß diese drei Prozesse beteiligt sind, wurde bisher nicht untersucht.

Der langsame Abbau der erfindungsgemäßen Wirkstoffe erlaubt es, die Wirkstoffe am Anfang des Klärprozesses zuzusetzen und eine wirksame Geruchsinhibierung über den ganzen Klärprozeß beizubehalten. Andererseits sind die entstehenden Produkte, nämlich die Phthalimidoalkylcarbonsäuren, mikrobiologisch leicht zu spalten und abzubauen, so daß dadurch keine weitere Umweltbelastung eintritt.

**Tabelle 1**

| Subjektive Geruchsbewertung im Prozeßabwasser einer Pappeherstellung nach Zugabe von PAP | | |
|---|---|---|
| Dosis g/kg | 15 min | 120 min |
| 0,050 | 0 | 1 |
| 0,200 | 1 | 2 |
| 1 | 2 | 3 |
| 10 | 3 | 3 |
| Bewertung: 0 = starker Geruch 1 = Faulgeruch 2 = wenig Geruch 3 = kein Geruch (neutraler Papiergeruch) | | |

## Patentansprüche

1. Verfahren zur biozitfreien Geruchsbekämpfung in Abwässern, **dadurch gekennzeichnet,** daß man dem Abwasser eine Phthalimidoalkylperoxicarbonsäure der Formel I, 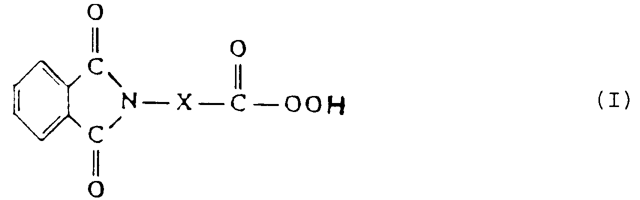 worin x eine Alkylengruppe mit 4 bis 10 Kohlenstoffen bedeutet,
in einer Konzentration von 0,1 bis 10 g/kg Abwasser zufügt.

2. Verfahren gemäß Anspruch 1, **dadurch gekennzeichnet,** daß die Wirksubstanz in einer Menge von 0,5 bis 5 g/kg Abwasser zugefügt wird.

3. Verfahren gemäß Anspruch 1 oder 2, **dadurch gekennzeich net,** daß die Wirksubstanz in einer Konzentration von 1 bis 2 g/kg Abwasser zugefügt wird.

4. Verfahren gemäß Anspruch 1, 2 oder 3, **dadurch gekennzeichnet,** daß die Wirkstoffe in Form ihrer Alkalisalze in wässriger Lösung, oder als wässrige Suspension zugefügt werden.

5. Verfahren gemäß einem der Ansprüche 1 bis 4, **dadurch gekennzeichnet,** daß als Wirkstoff Phthalimidohexanperoxicarbonsäure zugesetzt wird.
